# FASCICULE DE BREVET EUROPEEN

(11) **EP 1 325 735 B1**
(45) Date de publication et mention de la délivrance du brevet: **19.06.2013**
(21) Numéro de dépôt: 02368145.5
(22) Date de dépôt: 19.12.2002
(51) Int. Cl.: A61Q 17/04, A61K 8/44, A61K 8/64

(54) **Utilisation de dérivés de la L-arginine comme agents photoprotecteurs et optimisant la réponse mélanique dans des compositions cosmétiques**
Verwendung von L-Argininderivaten als Lichtschutzmittel und Förderer der Melaninsynthese in kosmetischen Zusammensetzungen
Use of L-arginine derivatives as photo-protecting agents and enhancing melanic response in cosmetic compositions

(30) Priorité: 04.01.2002 MC 2478
(43) Date de publication de la demande: 09.07.2003
(73) Titulaire: EXSYMOL S.A.M., MC-98000 Monte Carlo (MC)
(72) Inventeur: Seguin, Marie-Christine, 98000 Monaco (MC)
(74) Mandataire: McDade, Sophie V.G.A.

(56) Documents cités:
- FR-A- 2 710 340
- FR-A- 2 788 777
- US-A- 6 159 485
- US-B1- 6 245 342
- DATABASE CHEMICAL ABSTRACTS [en ligne] retrieved from STN Database accession no. 126: 176 685 XP002248166 & JP 08 337519 A (AJINOMOTO KK) 24 décembre 1996 (1996-12-24)

## Description

La présente invention a pour objet l'utilisation cosmétique de dérivés de la L-arginine comme agents photoprotecteurs et optimisant la réponse mélanique, tout particulièrement dans une composition visant à protéger la peau contre les effets néfastes du soleil.

Le rayonnement solaire induit au niveau de la peau une surproduction de pigments photoprotecteurs, les mélanines, par les mélanocytes. De nombreuses stratégies ont été décrites visant à modifier l'activité de ces cellules, et en particulier le fonctionnement d'une enzyme-clé de la mélanogénèse, la tyrosinase.
Cependant, les mécanismes impliqués dans ce processus de pigmentation cutanée se précisent peu à peu, et on sait aujourd'hui qu'une exposition de la peau aux radiations ultraviolettes (UV) induit la production de multiples facteurs endogènes modulant l'activité des mélanocytes. La découverte de ces agents, produits tant par les mélanocytes que les kératinocytes, a notamment conduit à la définition d'une entité fonctionnelle située dans les couches profondes de l'épiderme, l'unité "mélano-épidermique" (T.D. Fitzpatrick, Biology of the melanin pigmentary system (1979), T.D. Fitzpatrick Ed, McGraw Hill Book Co, N.Y., pp.131-163) où s'effectue une coopération cellulaire entre kératinocytes et mélanocytes.

Il est désormais acquis que le monoxyde d'azote (NO) est un de ces agents endogènes capables d'activer la mélanogénèse, via son action sur l'unité "mélano-épidermique". Des études ont ainsi démontré qu'en réponse à une stimulation par les UV, d'une part, les mélanocytes réagissent en produisant du NO endogène qui induit une sur-expression de la tyrosinase (M. Sasaki, Pigment Cell Res., vol.13 (2000), pp.248-252). D'autre part, les kératinocytes produisent également du NO qui lui aussi active la synthèse des pigments mélaniques via cet effet de communication cellulaire entre mélanocytes et kératinocytes adjacents (C. Roméro-Graillet, J. Clin. Invest., vol.99 (1997), pp.635-642).

Aussi, il existe un besoin d'agir sur cette unité "mélano-épidermique", en particulier sur la coopération cellulaire au sein de cette unité, et donc d'agir sur la production de NO.

Cependant, le NO est depuis longtemps considéré comme peu compatible avec une utilisation cosmétique en raison de la multiplicité de ses effets biologiques (acteur important de la réponse inflammatoire, vasodilatateur, neuromédiateur) et notamment destructeurs (cytotoxique, générateur *in vivo* de radicaux libres et de sous-produits toxiques tels que l'anion peroxynitreux, inducteur de l'apoptose).
Toutefois, des travaux plus récents ont souligné les propriétés protectrices de NO avec un effet antioxydant sur une peau soumise à des UV-B (S.C. Lee, British J. of Dermatology, vol. 142 (2000), pp.653-659), et surtout un rôle de messager biologique essentiel à de nombreux processus physiologiques utiles.

Cette dualité des effets de NO s'explique en grande partie par la co-existence dans les tissus, et notamment les tissus cutanés, des deux formes différentes de l'enzyme spécialisée dans la production de NO, la NO-synthase (NOS). On distingue en effet l'isoforme inductible de la NO-synthase (iNOS), présente dans les tissus que sous certaines conditions, de l'isoforme constitutive (cNOS) toujours présente dans les tissus. Ces deux isoformes ont des rôles biologiques différents.

Le problème posé est donc de renforcer la protection de la peau contre les effets néfastes du soleil en agissant sur le mécanisme NO-dépendant de sa réponse mélanique, tout en tenant compte de la situation physiologique précédemment décrite, à savoir la dualité des effets de NO, et le fait que l'induction d'effets secondaires indésirables est inacceptable pour un produit cosmétique.

La nouvelle approche proposée dans ce brevet repose donc sur l'utilisation d'actifs optimisant la réponse mélanique à la suite d'un stimulus UV, en favorisant la production de NO dans l'épiderme, sans pour autant induire une production anormale de NO par les cellules cutanées. Identiquement, une autre caractéristique importante de l'invention est d'éviter d'éventuels effets secondaires indésirables pouvant résulter d'une production anormale de NO par ces cellules cutanées en l'absence d'une stimulation par les UV.

Dans la perspective de fournir à la NO-synthase de la "matière première", l'arginine sous sa forme L (L-ARG) apparait comme une solution audit problème, puisqu'il s'agit du substrat naturel de ladite NO-synthase, et est considéré comme le précurseur naturel de NO.

Il est important de souligner que ce rôle de précurseur est bien distinct de celui d'activateur ou d'inducteur, par exemple, du degré d'expresion de l'enzyme et, qu'en aucun cas, un apport d'arginine ne peut induire une surproduction de NO.

La faible biodisponibilité de l'arginine déposée à la surface de la peau constitue toutefois un obstacle majeur pour une utilisation de l'arginine aux fins revendiquées, alors que l'unité "mélano-épidermique" est située dans les couches profondes de l'épiderme. En effet, sa faible pénétration cutanée, conséquence d'une structure très polaire, et sa métabolisation par les couches supérieures de la peau s'opposent à son utilisation par l'unité "mélano-épidermique".

L'objet de la présente invention est par conséquent de proposer l'utilisation de dérivés d'arginine, capables de reformer *in vivo* un substrat de la NO-synthase, et possédant une biodisponibilité compatible avec une stimulation de l'unité "mélano-épidermique". Une autre caractéristique de l'utilisation de ces dérivés selon l'invention est de réduire la métabolisation de la L-arginine par les couches superficielles de l'épiderme.

Par cet apport exogène de L-arginine sous la forme de dérivés d'arginine selon l'invention, il est permis d'optimiser le fonctionnement de mécanismes cutanés NO-dépendants, en réponse à une exposition de la peau au rayonnement solaire. En effet, les UV provoquent, tant au niveau des kératinocytes que des mélanocytes, l'apparition de la forme inductible de la NO-synthase (iNOS) qui ne produit, sur l'arginine naturellement présente dans la peau, qu'une quantité physiologique de NO insuffisante. Un apport d'arginine exogène devient donc nécessaire pour un bon fonctionnement de l'unité "mélano-épidermique" puisque, chez les mammifères, pratiquement toutes les cellules utilisent de l'arginine d'origine extra-cellulaire (S.M. Morris Jr, Annu. Rev. Nutr., vol.12 (1992), pp.81-101). Ce même problème a d'ailleurs été évoqué pour d'autres situations telles le processus de cicatrisation (J-H. Chyun, J. of Nutrition, vol.114 (1984), pp.1697-1704) ou certaines situations pathologiques (M.A. Creager, J. Clin. Invest., vol.90 (1992), pp.1248-1253).

En outre, il est connu que les isoformes dites "constitutives" de la NO-synthase ne réagissent pas à un apport exogène d'arginine. Elles ont en effet besoin d'être pré-activées par d'autres agents tels que le calcium et la calmoduline. Par conséquent, une nouvelle fois, un apport exogène biodisponible selon l'invention ne peut pas induire, en l'absence d'une stimulation par les UV, une production indésirable de NO au niveau de la peau.

Par ailleurs, il est aussi important de noter que mélanocytes et kératinocytes expriment la iNOS en réponse aux UV. Les conditions physiologiques favorables à l'utilisation des dérivés selon l'invention sont donc réunies puisque ces dérivés pourront alors agir sur les deux constituants de l'unité "mélano-épidermique" et qu'ils pourront réellemment favoriser l'activité de celle-ci.

Une autre avantage relatif à l'utilisation des dérivés d'arginine, objets de l'invention, est lié à leur pouvoir photoprotecteur, notamment en raison de leur caractère antioxydant. Il est bien connu en effet que le rayonnement solaire est une cause majeure de stress oxydatif au niveau cutané et notamment au niveau des mélanocytes, particulièrement vulnérables. Les protéger avec l'utilisation des dérivés d'arginine selon l'invention revient à agir sur la réponse mélanique et notamment à favoriser la production de mélanine. En outre, le pouvoir protecteur de ces dérivés est complété par les effets connus (comportement chélateur vis à vis d'ions métalliques, capacité à former *in situ* des agents photoprotecteurs que sont les nitroso-thiols) des sous-produits issus de leur biotransformation, notamment de la L-arginine.

A ce jour, et à notre connaissance, aucune utilisation de l'arginine ou de dérivés d'arginine n'a été revendiquée comme acteur de la pigmentation cutanée, et en particulier du processus d'hyperpigmentation induit par le soleil.

Au contraire, la demande de brevet japonais publiée sous le N° JP 3 178 912 A2 préconise l'emploi d'arginine et de certains de ses dérivés comme agent de blanchiment.

L'association d'un dérivé d'arginine, le N^{ω}-monométhyl-L-arginine acétate, à des filtres solaires dans le but de renforcer la protection contre les effets négatifs du rayonnement solaire a été rapportée (G.M. Halliday, Redox Rep., vol. 4(6) (1999), pp. 316-318). Ce dérivé n'entre pas dans le champ de l'invention et surtout il diffère des objets de l'invention par son principe d'action, dans la mesure où il s'agit d'un inhibiteur de NO-synthase et qu'il ne reforme pas non plus de la L-arginine au contact de la peau. De même avec une séquence comprenant au moins quatre acides aminés analogue de l'hormone MSH (« Mélanocyte Stimulating Hormone »), les peptides décrits dans la demande de brevet FR 2 710 340 n'interfèrent pas avec la portée de la présente invention, l'activité revendiquée par ces dérivés peptidiques, dans le cas d'un effet sur la mélanogénèse, ne s'exprimant que par une action sur l'enzyme tyrosinase.

Par ailleurs, la demande de brevet WO 00/44368 fait état d'agents capables d'activer la production de NO endogène et de stimuler la mélanogénèse. Il s'agit de diols terpéniques capables, selon différentes études publiées par les mêmes auteurs (D.A. Brown, J. Invest. Dermatol., vol.110 (1998), pp.428-437 et D.A. Brown, Pigment Cell Res., vol.12 (1999), pp.36-47), de provoquer la synthèse de mélanine en l'absence de rayonnement solaire. Dans une approche d'"auto-bronzage", les effets du soleil sont reproduits par l'utilisation de ces actifs, mais ceci n'est pas l'effet revendiqué dans la présente invention.

La société Beiersdorf propose l'application d'arginine par voie topique pour produire ***in situ*** un agent photoprotecteur de type nitroso-thiol (G. Sauermann, Proc. of the 19th IFSCC Congress (1996), R089). Cette application ne concerne pas un processus de pigmentation photo-induit et notamment une optimisation de la réponse mélanique.

Des dérivés lipophiles d'arginine sont décrits (T. Cavaletti "Lipoaminoacids are powerful scavengers of free radicals" Proc. of the 16th IFSCC Congress, vol.2 (1990), pp.354-363) et proposés comme agent anti-radicalaire, mais ils ne peuvent reformer ***in situ*** l'aminoacide de départ.

A notre connaissance, aucun dérivé antioxydant de l'art antérieur possèdant ces propriétés n'a été utilisé pour agir sur la réponse mélanique UV-induite. Certes, on trouve encore des dérivés prodrogues à base d'arginine puisque capables de reformer ***in vivo*** de l'arginine, mais dans un contexte où l'arginine n'est pas le principe actif (H. Tsunematsu "Synthesis and enzymatic hydrolysis of aspirin-basic aminoacid ethyl esters" Int. J. Pharmaceutics, vol. 68 (1991), pp.77-86). On trouve également une séquence peptidique à base d'arginine dans la demande de brevet FR 2 788 777, mais elle ne vise aucune action sur la réponse mélanique. De même, les brevets JP 08337519 (Chemical Abstract 126 : 176685) et US 6,159,485 divulguent respectivement des composés de type N-acyl-arginine et N-acétyl-arginine, mais dans ces documents aucune action sur la réponse mélanique n'est visée.

Enfin, le mode d'action des dérivés objets de l'invention doit enfin être clairement distingué de celui d'une famille de composés dits "donneurs de NO" (ex. : S-nitroso-N-acetylpenicillamine, 1-propamine,3-(2-hydroxy-2-nitroso-1-propylhydrazine) dont l'effet sur la mélanogénèse a été décrit (A.A. Oureshi, J. Invest. Dermatol., vol.108, IV (1997), pp.627). Ces agents produisent du NO sans utiliser les systèmes biologiques que sont les NO-synthases, les co-facteurs, ... . Ce principe d'action est bien différent de celui de l'invention et est incompatible avec une utilisation en cosmétique.

La famille de dérivés de l'arginine, objets de la présente invention, a déjà été décrite en partie dans une demande de brevet européen déposée par la demanderesse et publiée sous le N° EP 1 060 739 A1. Celle-ci décrit en effet une composition cosmétique amincissante par voie topique à base de dérivés d'arginine.

Les récentes expériences réalisées par la demanderesse ont montré que ces mêmes dérivés pouvaient être utilisés comme agents capables d'optimiser la réponse mélanique et donc de protéger la peau contre les effets néfastes du soleil, via également leurs propriétés protectrices intrinsèques.

Bien que les différentes isoformes de NO-synthase présentent une forte spécificité vis-à-vis de l'arginine, il a été démontré (C. Moali et al., Biochemistry, 37(29) (1998), pp.10453) qu'elles pouvaient tout de même accepter d'autres substrats, et notamment un composé analogue de la L-arginine, l'homo-L-arginine.

C'est pourquoi la présente invention a pour objet l'utilisation cosmétique d'une famille de dérivés de la L-arginine ou l'un quelconque de leurs sels, comme agents photoprotecteurs et optimisant la réponse mélanique, lesdits dérivés étant mentionnés à la revendication 1.

Tous ces dérivés possèdent une biodisponibilité améliorée avec un caractère lipophile propre à favoriser leur pénétration cutanée, et des caractéristiques structurales qui s'opposent à leur métabolisation par les enzymes pouvant utiliser l'arginine (NO-synthases, arginases).
Ceci est le plus simplement réalisé par estérification de la fonction α-carboxylique et/ou amidification de la fonction α -amine, transformations qui rendent, d'une part, ces fonctions non ionisables à pH physiologique et qui renforcent le caractère lipophile, et, d'autre part, les dérivés obtenus insensibles à l'action des NO-synthases et des arginases.

Il est aussi utile que les radicaux R₁ et R₂ soient des substituants biodégradables, pouvant être hydrolysés aux conditions de pH physiologique ou en présence d'enzymes cutanées pour reformer *in vivo* de l'arginine, ou son analogue.

La NO-synthase inductible étant sensible à la configuration de son substrat, il est essentiel que, dans tous les composés selon l'invention, la configuration du carbone asymétrique de l'arginine (ou stéréochimie) soit identique à celle de l'arginine utilisable par la NO-synthase (forme L).

Un autre mode de réalisation particulièrement avantageux de l'invention consiste à substituer les fonctions α-carboxylique et/ou α-amine de l'arginine, par des radicaux eux-mêmes hydrophobes qui contribueront à renforcer le caractère lipophile de la molécule précurseur, et donc à favoriser encore plus la pénétration de l'actif dans l'épiderme.
Ceci est obtenu lorsque la fonction α-amine de l'arginine est substituée par un radical peu polaire, comme le groupement acétyle, ou par un substituant plus hydrophobe tel qu'un radical propionyle ou *tert*-butyloxycarbonyle (Boc). Ceci est également obtenu lorsque la fonction α-carboxylique de l'arginine est estérifiée par un radical ethyle, ou par un substituant encore plus hydrophobe tel que le radical isopropyle, *n*-butyle, *n*-octyle, ou encore par le pivalloyloxymethyle, substituant qui a la particularité d'être stable au pH externe de la peau (pH 5 environ) et hydrolysé à pH proche de la neutralité, et qui est le pH des couches plus profondes de la peau.

Dans la perspective d'obtenir un dérivé le plus lipophile posible, lorsque le radical est un α-aminoacide, il est préférable de subtituer sa fonction α-carboxylique libre de façon à la rendre non ionisable à pH physiologique.

Selon un autre mode de réalisation permettant d'augmenter la résistance du composé aux systèmes enzymatiques cutanés, les α-aminoacides utilisés pour former les radicaux sont choisis parmi les aminoacides substrats d'enzymes spécifiques. Ces dérivés rencontreront donc sur leur passage dans les couches supérieures de la peau moins d'enzymes capables de les biotransformer. De tels aminoacides, substitués ou non sur leurs fonctions α-amine ou α -carboxylique, peuvent être la citrulline, l'homosérine, la norleucine, la norvaline, l'ornithine, la penicillamine ou la sarcosine.
Selon ce mode de réalisation, un dérivé particulièrement intéressant est le dipeptide naturel L-citrullinyl-L-arginine produit par une algue rouge (*Chondrus crispus*)

Enfin, un mode de réalisation particulièrement intéressant de l'invention consiste à réaliser des dérivés dont le potentiel photoprotecteur est aussi élevé que possible. Selon ce mode de réalisation particulier de l'invention, on condense sur la L-arginine, un substituantbiodégradable propre à renforcer tout particulièrement le pouvoir antioxydant du dérivé.

L'objet de l'invention est le dérivé où l'arginine, est condensé à l'aminoacide L-tyrosine substitué ou non sur sa fonction α-amine ou α -carboxylique. En effet, cet aminoacide possède, en plus des caractéristiques déjà évoquées (hydrophobe, antioxydant), la propriété d'être le substrat de la tyrosinase, une enzyme-clé dans la biosynthèse des mélanines. Ce mode de réalisation particulier de l'invention présente donc la particularité de pouvoir favoriser la pigmentation cutanée UV-induite, en agissant sur deux activités enzymatiques nécessaires à la réponse mélanique : l'activité de la NO-synthase et l'activité de la tyrosinase, et en apportant à ces enzymes leurs substrats respectifs.

Les composés peuvent être préparés suivant des méthodes connues de l'homme du métier.

Les exemples qui suivent donnent une liste non limitative des derivés selon l'invention, et illustrent de façon non limitative les modes d'utilisation possibles desdits derivés.

### Exemples de structure :

### N-acetyl-L-arginine ethyl ester

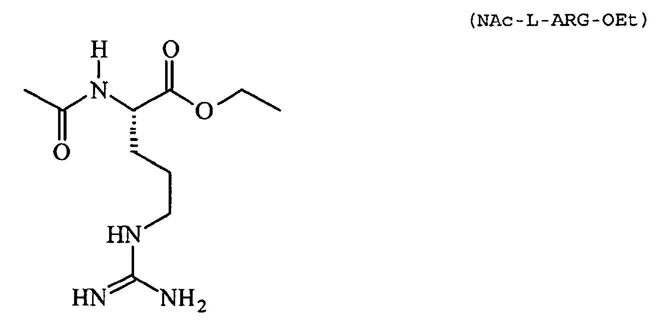

### N-acetyl-L-tyrosyl-L-arginine ethyl ester

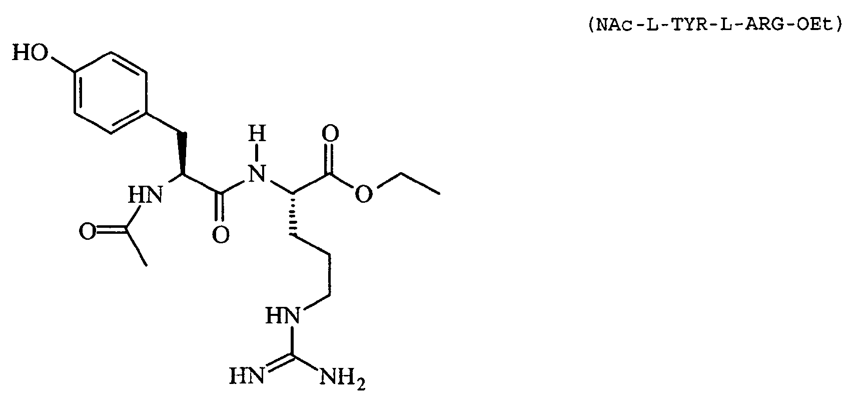

### N-acetyl-L-arginyl-(D ou L)-isoleucine ethyl ester

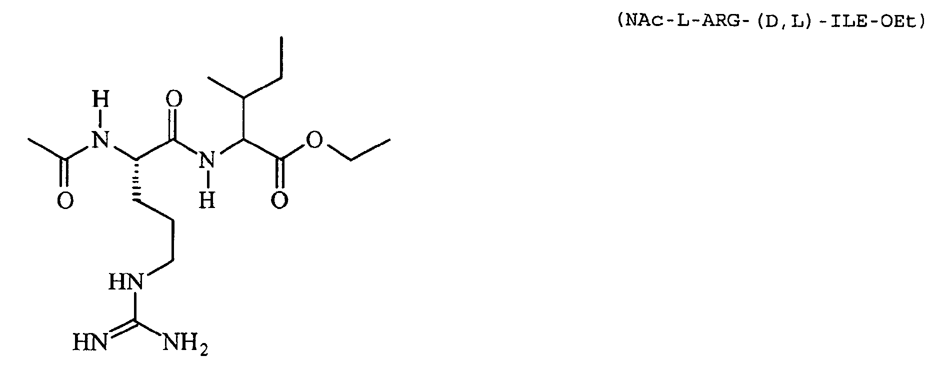

### N-acetyl-(D ou L)-histidyl-L-arginine ethyl ester

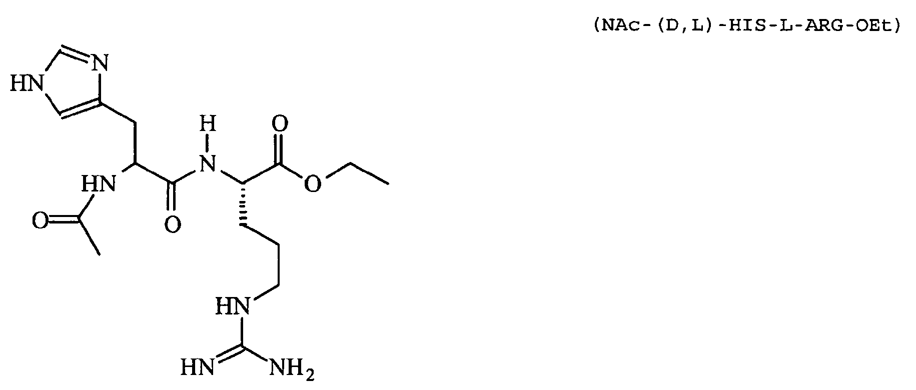

### N-acetyl-(D ou L)methionyl-L-arginine ethyl ester

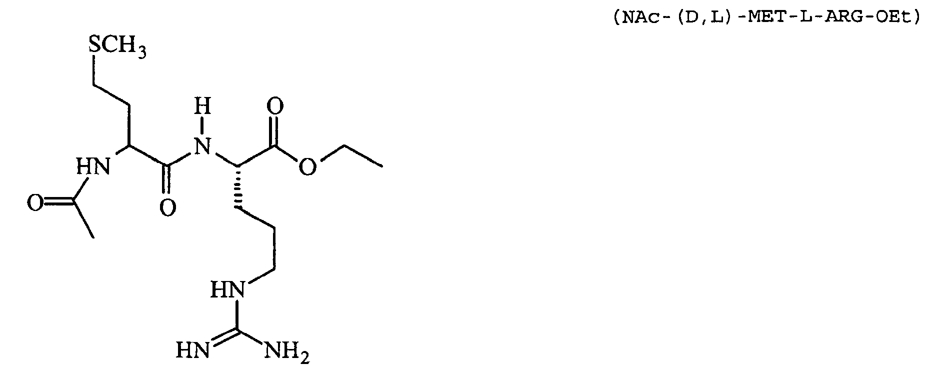

### N-acetyl-L-citrullinyl-L-arginine ethyl ester

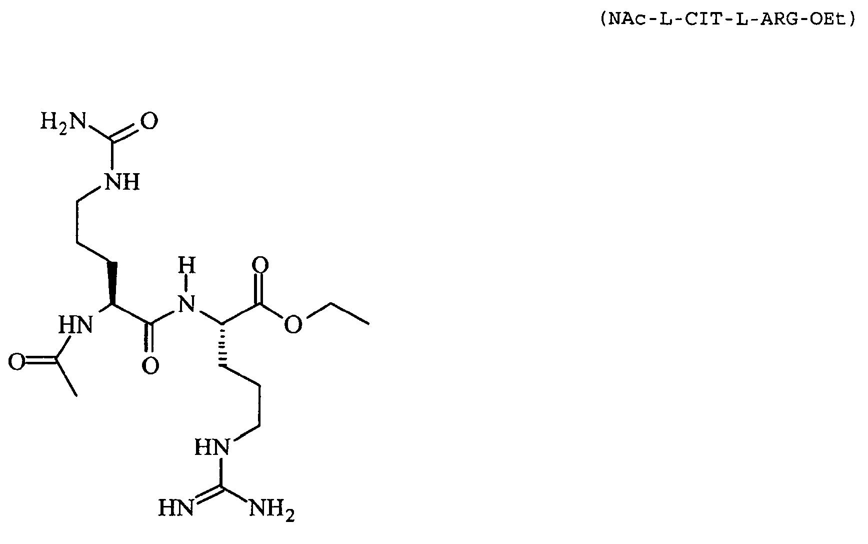

### N-Acetyl-(D ou L)-phenylalanyl-L-arginine octyl ester

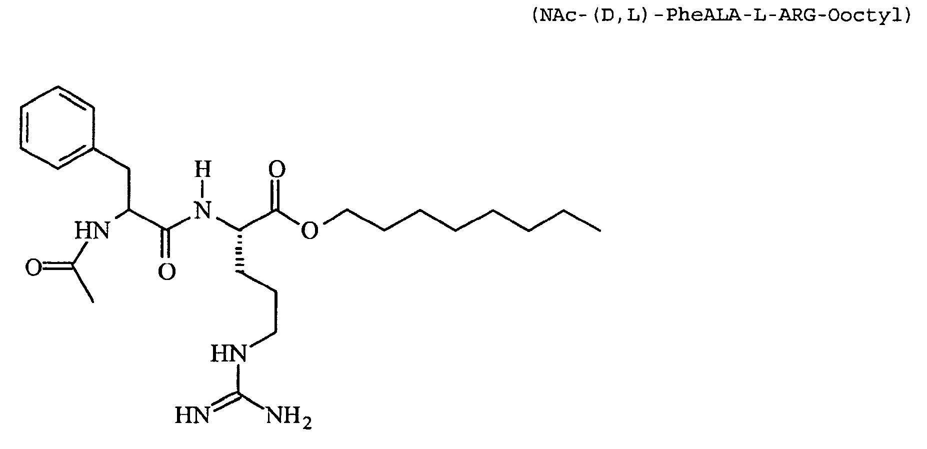

### N-Boc-L-citrullinyl-L-arqinine ethyl ester

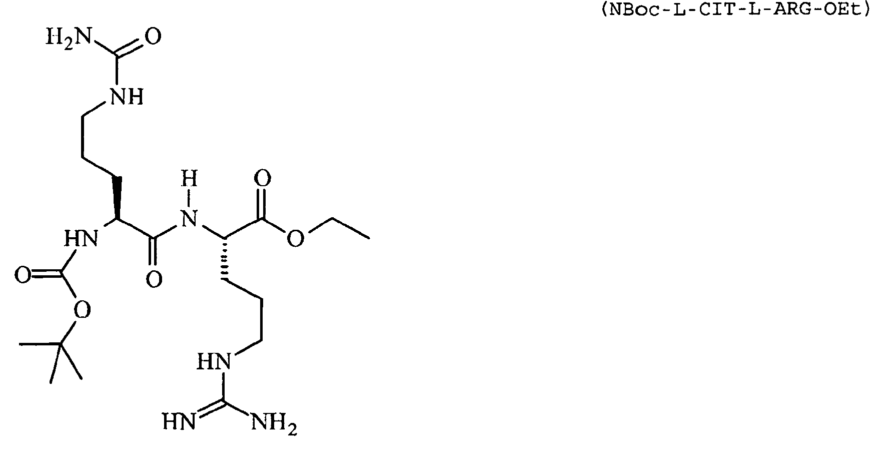

### Exemples d'activité :

### Test 1 : démonstration du pouvoir protecteur des dérivés d'arginine (mesure du piègeage du radical hydroxyle OH•).

Le test, réalisé selon la méthode décrite par B. Halliwell et coll. (Analytical Biochem., vol. 165 (1987), pp.215-219), met en évidence la protection du deoxyribose par la substance testée (S) contre les effets de OH•. Le pouvoir protecteur est déduit de la comparaison des cinétiques de réaction, exprimées par une constante de vitesse de S sur OH• (piégeage de OH•).

### Résultats:

| substance S | constante de vitesse de piégeage (M⁻¹.S⁻¹) |
|---|---|
| L-arginine | 1,5 (±0,2)x10⁹ |
| NAc-L-ARG-OEt | 5,2 (±0,2)x10⁹ |
| NAc-L-ARG-L-ILE-OEt | 6,1 (±0,4)x10⁹ |
| NAc-L-HIS-L-ARG-OEt | 9,0 (±0,2)x10⁹ |
| L-histidine | 2,5 (±0,2)x10⁹ |
| NAc-(D,L)-MET-L-ARG-OEt | 0,7 (±0,2)x10¹⁰ |
| NAc-L-TYR-L-ARG-OEt | 8,1 (±0,3)x10⁹ |

Les constantes de vitesse correspondent à la valeur moyenne (± D.S.) calculée à partir de 3 expériences indépendantes

### Test 2 : démonstration de l'effet des dérivés d'arginine sur l'unité "mélano-épidermique" et la réponse mélanique

Nous avons mis au point un modèle expérimental reproduisant l'unité "mélano-épidermique", et qui permet de mettre en évidence les effets d'un apport exogène de dérivés d'arginine sur la réponse mélanique à la suite de rayonnements U.V. (effet direct sur les mélanocytes et indirect via la "communication cellulaire" entre kératinocytes et mélanocytes).
Il s'agit d'un modèle de co-culture *in vitro* de mélanocytes et de kératinocytes (voir représentation schématique du modèle), possédant un milieu de culture (MdC) particulier.

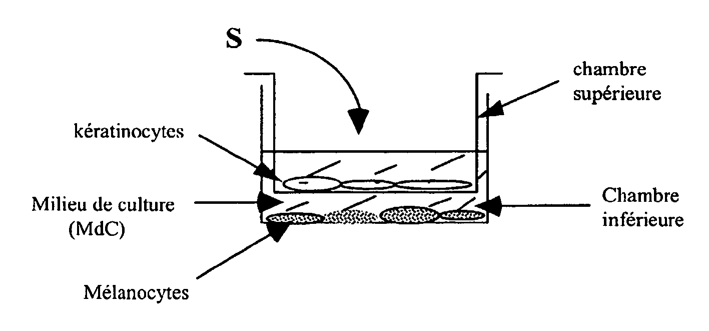

Le fond poreux de la chambre supérieure permet le passage, d'un compartiment à l'autre, des facteurs endogènes sécrétés par les cellules (NO) et la substance (S).

### Résultats :

| **Substance S** ajoutée à la co-culture | co-culture + MdC "ARG = 84mg/L"^{a}sans irradiation | co-culture + MdC "ARG = 84mg/L"^{a} irradiée par UVB | co-culture + MdC "ARG-free"^{b} irradiée par UVB |
|---|---|---|---|
| 0 (sans ajout) | 22,0 | 40,6 | 26,4 |
| | (±0,5) | (±5) | (±3,3) |
| NAc-ARG-OEt (0,48 mM) | 24,4 | 38,4 | 43,8 |
| | (±1,2) | (±4,5) | (±5,1) |
| NAc-ARG-OEt (0,24 mM) | 20,9 | 42,6 | 37,5 |
| | (±2,3) | (±3,2) | (±2,2) |
| NAc-ARG-OEt (0,12 mM) | 23,1 | 39,5 | 30,7 |
| | (±0,8) | (±3,4) | (±3,1) |
| NAc-HIS-ARG-OEt (0,48 mM) | 18,8 | 40,7 | 42,5 |
| | (±0,8) | (±4) | (±4,9) |
| NAc-HIS-ARG-OEt (0,24 mM) | 22,5 | 36,2 | 41,0 |
| | (±1,0) | (±2,5) | (±1,5) |
| NAc-HIS-ARG-OEt (0,12 mM) | 22,7 | 41,6 | 38,8 |
| | (±0,8) | (±2,5) | (±2,3) |

| | | | |
|---|---|---|---|
| a: le milieu de culture contient des concentrations supra-physiologiques d'arginine (MdC standard) | | | |
| b: le milieu de culture reproduit une concentration physiologique en arginine insuffisante | | | |

## Revendications

1. Utilisation d'au moins un dérivé de la L-arginine, ou l'un quelconque de ses sels, dans la préparation d'une composition cosmétique comme agent optimisant la réponse mélanique par augmentation de celle-ci à la suite d'un stimulus U.V., en favorisant la production d'oxyde nitrique (NO) dans l'épiderme, sans pour autant induire une production anormale de NO par les cellules cutanées, ledit dérivé étant choisi parmi le N-acétyl-L-arginine éthyl ester, le N-acétyl-L-tyrosyl-L-arginine éthyl ester, le N-acétyl-L-arginyl-(D, L)-isoleucine éthyl ester, le N-acétyl-(D, L)-histidyl-L-arginine éthyl ester, le N-acétyl-(D, L)methionyl-L-arginine éthyl ester, le N-acétyl-L-citrullinyl-L-arginine éthyl ester, le N-acétyl-(D, L)-phénylalanyl-L-arginine octyl ester, le N-Boc-L-citrullinyl-L-arginine éthyl ester.

2. Utilisation selon la revendication 1, **caractérisée en ce que** le dérivé de L-arginine est le N-acétyl-L-arginine éthyl ester.

3. Utilisation selon la revendication 1, **caractérisée en ce que** le dérivé de L-arginine est le N-acétyl-L-tyrosyl-L-arginine éthyl ester.

4. Utilisation selon la revendication 1, **caractérisée en ce que** le dérivé de L-arginine est le N-acétyl-L-arginyl-(D, L)-isoleucine éthyl ester.

5. Utilisation selon la revendication 1, **caractérisée en ce que** le dérivé de L-arginine est le N-acétyl-(D, L)-histidyl-L-arginine éthyl ester.

6. Utilisation selon la revendication 1, **caractérisée en ce que** le dérivé de L-arginine est le N-acétyl-(D, L)methionyl-L-arginine éthyl ester.

7. Utilisation selon la revendication 1, **caractérisée en ce que** le dérivé de L-arginine est le N-acétyl-L-citrullinyl-L-arginine éthyl ester.

8. Utilisation selon la revendication 1, **caractérisée en ce que** le dérivé de L-arginine est le N-Acétyl-(D,L)-phénylalanyl-L-arginine octyl ester.

9. Utilisation selon la revendication 1, **caractérisée en ce que** le dérivé de L-arginine est le N-Boc-L-citrullinyl-L-arginine éthyl ester.

## Patentansprüche

1. Eine Verwendung von mindestens einem Derivat von L-Arginin oder einem beliebigen seiner Salze bei der Herstellung einer Kosmetikzusammensetzung als Agens zur Optimierung der Melaninreaktion durch Verstärkung derselben infolge eines UV-Stimulus, indem die Produktion von Stickstoffmonoxid (NO) in der Epidermis unterstützt wird, ohne dass jedoch eine anormale Produktion von NO durch die Hautzellen induziert wird, wobei das Derivat ausgewählt ist aus N-Acetyl-L-arginin-ethylester, N-Acetyl-L-tyrosyl-L-arginin-ethylester, N-Acetyl-L-arginyl-(D,L)-isoleucin-ethylester, N-Acetyl-(D,L)-histidyl-L-arginin-ethylester, N-Acetyl-(D,L)methionyl-L-arginin-ethylester, N-Acetyl-L-citrullinyl-L-arginin-ethylester, N-Acetyl-(D,L)-phenylalanyl-L-arginin-octylester, N-Boc-L-citrullinyl-L-arginin-ethylester.

2. Verwendung gemäß Anspruch 1, **dadurch gekennzeichnet, dass** das Derivat von L-Arginin N-Acetyl-L-arginin-ethylester ist.

3. Verwendung gemäß Anspruch 1, **dadurch gekennzeichnet, dass** das Derivat von L-Arginin N-Acetyl-L-tyrosyl-L-arginin-ethylester ist.

4. Verwendung gemäß Anspruch 1, **dadurch gekennzeichnet, dass** das Derivat von L-Arginin N-Acetyl-L-arginyl-(D,L)-isoleucin-ethylester ist.

5. Verwendung gemäß Anspruch 1, **dadurch gekennzeichnet, dass** das Derivat von L-Arginin N-Acetyl-(D,L)-histidyl-L-arginin-ethylester ist.

6. Verwendung gemäß Anspruch 1, **dadurch gekennzeichnet, dass** das Derivat von L-Arginin N-Acetyl-(D,L)methionyl-L-arginin-ethylester ist.

7. Verwendung gemäß Anspruch 1, **dadurch gekennzeichnet, dass** das Derivat von L-Arginin N-Acetyl-L-citrullinyl-L-arginin-ethylester ist.

8. Verwendung gemäß Anspruch 1, **dadurch gekennzeichnet, dass** das Derivat von L-Arginin N-Acetyl-(D,L)-phenylalanyl-L-arginin-octylester ist.

9. Verwendung gemäß Anspruch 1, **dadurch gekennzeichnet, dass** das Derivat von L-Arginin N-Boc-L-citrullinyl-L-arginin-ethylester ist.

## Claims

1. Use of at least one derivative of L-arginine, or any of their salts, in the manufacture of a cosmetic composition as an agent enhancing the melanic response by increasing it following a U.V. stimulus, while promoting nitric oxide production (NO) within the epidermis but without inducing any abnormal NO production by skin cells, said derivative being chosen among N-acetyl-L-arginine ethyl ester, N-acetyl-L-tyrosyl-L-arginine ethyl ester, N-acetyl-L-arginyl-(D,L)-isoleucine ethyl ester, N-acetyl-(D,L)-histidyl-L-arginine ethyl ester, N-acetyl-(D,L)-methionyl-L-arginine ethyl ester, N-acetyl-L-citrullinyl-L-arginine ethyl ester, N-acetyl-(D,L)-phenylalanyl-L-arginine octyl ester, N-Boc-L-citrullinyl-L-arginine ethyl ester.

2. Use according to claim 1, **characterized in that** the derivative of L-arginine is N-acetyl-L-arginine ethyl ester.

3. Use according to claim 1, **characterized in that** the derivative of L-arginine is N-acetyl-L-tyrosyl-L-arginine ethyl ester.

4. Use according to claim 1, **characterized in that** the derivative of L-arginine is N-acetyl-L-arginyl-(D,L)-isoleucine ethyl ester.

5. Use according to claim 1, **characterized in that** the derivative of L-arginine is N-acetyl-(D,L)-histidyl-L-arginine ethyl ester.

6. Use according to claim 1, **characterized in that** the derivative of L-arginine is N-acetyl-(D,L)-methionyl-L-arginine ethyl ester.

7. Use according to claim 1, **characterized in that** the derivative of L-arginine is N-acetyl-L-citrullinyl-L-arginine ethyl ester.

8. Use according to claim 1, **characterized in that** the derivative of L-arginine is N-acetyl-(D,L)-phenylalanyl-L-arginine octyl ester.

9. Use according to claim 1, **characterized in that** the derivative of L-arginine is N-Boc-L-citrullinyl-L-arginine ethyl ester.
